# EUROPEAN PATENT APPLICATION

(11) **EP 4 467 207 A1**
(43) Date of publication of application: **27.11.2024**
(21) Application number: 24177902.4
(22) Date of filing: 24.05.2024
(51) Int. Cl.: A63B 27/02, A61F 5/01

(54) **LEG HARNESS AND LOCKING MECHANISM DEVICE**

(30) Priority: 24.05.2023 US 202363468642 P; 07.11.2023 US 202318503352
(71) Applicant: Cassler, Ian, Mt. Clemens, MI 48043 (US)
(72) Inventor: Cassler, Ian, Mt. Clemens, MI 48043 (US)
(74) Representative: DREISS Patentanwälte PartG mbB

(57) **Abstract**

A leg harness and locking mechanism device, which can be attached to a leg of a user, such as a climber of a wooden structure, and locked at one or both of the user's knees to relieve pressure on the knee or knees during work, may include at least one harness assembly. The harness assembly may be deployed on a leg of a user. The harness assembly may include a hinge which facilitates pivoting or folding of the assembly at the corresponding knee of the user. The harness assembly may have a hinge locking mechanism which is selectively operable to lock the hinge and prevent the harness assembly from pivoting or folding at the knee of the user, thereby relieving pressure on the user's knee or knees. The hinge locking mechanism may further be operable to unlock the hinge and facilitate folding of the harness assembly at the knee.

## Description

### Cross-Reference to Related Application

This application claims the benefit of United States Provisional Patent Application No. 63/468,642, filed on May 24, 2023, which is incorporated herein by reference in its entirety.

### Field of the Invention

The present invention relates generally to leg harnesses used in climbing wooden structures, and more particularly, to a leg harness and locking mechanism device, which can be attached to a leg of a user, such as a climber of a wooden structure, and locked at one or both of the climber's knees to relieve pressure on the knees during work.

### Background of the Invention

In some cases, it may be necessary or desirable for work persons, such as arborists, electrical line technicians, or other climbers, to climb wooden structures such as trees and telephone poles. For example, pruning is a practice which involves the selective removal of certain parts of a plant, such as branches, buds, or roots. The practice entails the targeted removal of diseased, damaged, dead, non-productive, structurally unsound, or otherwise unwanted plant material from crop and landscape plants. In these cases, an arborist may climb a tree to saw or cut off the damaged or diseased branches on the tree. Electrical line technicians may climb telephone poles to replace or maintain transformers, electrical cables, or the like.

The safety of climbers is of paramount importance as such persons climb wooden structures. Therefore, various safety measures have been devised for such persons to wear during climbing. For example, climbing spurs are a common tool for tree removal operations. Climbing spurs may be provided on leg harnesses which attach to the legs of the climber and include spikes or gaffs which engage the structure to enable the climber for an optimal position to make a cut in the structure. Various types of leg harnesses with climbing spurs may be available depending on the climbing application.

There is need for a leg harness and locking mechanism device which can be attached to a leg of a climber of a wooden structure and locked at the climber's knees to relieve pressure on the knees during work.

### Summary of the Invention

The present invention is directed to a leg harness and locking mechanism device which can be attached to a leg of a user, such as a climber of a wooden structure, and locked at one or both of the user's knees to relieve pressure on the knee or knees during work. The leg harness device may include at least one harness assembly. The harness assembly may be suitably configured for deployment on at least one of the right leg and the left leg of a user in preparation for climbing a wooden structure. The harness assembly may include a hinge which facilitates pivoting or folding of the assembly at the corresponding knee of the user. The harness assembly may have a hinge locking mechanism which is selectively operable to lock the hinge and maintain the harness assembly in a straight position to prevent the harness assembly from pivoting or folding at the knee of the user. Accordingly, the locked hinge may relieve pressure on the user's knee or knees. The hinge locking mechanism may further be operable to selectively unlock the hinge and facilitate folding or pivoting of the harness assembly at the knee or knees of the user.

In an illustrative implementation of the invention, a leg harness and locking mechanism device which can be attached to a leg of a user and locked at one or both of the user's knees to relieve pressure on the knee or knees during work may include a right harness assembly and a left harness assembly. The right harness assembly and the left harness assembly may be suitably configured for deployment on the respective right and left legs of a user in preparation for climbing a wooden structure. Each of the right harness assembly and the left harness assembly may include a hinge which facilitates pivoting or folding of the assembly at the corresponding knee of the user. At least one of the right harness assembly and the left harness assembly may include a hinge locking mechanism which is selectively operable to lock the hinge and maintain the harness assembly in a straight position, preventing the harness assembly from pivoting or folding at the knee of the user. The hinge locking mechanism may further be operable to unlock the hinge and facilitate folding or pivoting of the assembly at the user's knee. The hinge locking mechanism may have a hinge locking arm configured to detachably engage the hinge. The hinge locking arm may be selectively deployable in a locking position and an unlocking position. In the locking position, the hinge locking arm may engage the hinge and lock the hinge to maintain the harness assembly in the straight position and prevent the harness assembly from pivoting or folding at the knee of the user. The locked hinge may relieve pressure on the user's knee or knees. In the unlocking position, the hinge locking arm may disengage the hinge to unlock the hinge and facilitate folding or pivoting of the harness assembly at the knee or knees of the user.

In a second aspect, the same harness assembly may be configured for use as the right harness assembly for deployment on the right leg of the user or as the left harness assembly for deployment on the left leg of the user.

In another aspect, right and left harness assemblies may be configured for deployment on the respective right and left legs of the user.

In another aspect, each harness assembly may be configured for deployment on the outside or inside of the corresponding leg of the user.

In another aspect, each of the right harness assembly and the left harness assembly may include an frame which is securable on the corresponding right or left leg of the user. The hinge may facilitate pivoting of the frame at the knee of the user.

In another aspect, the frame of the right and left harness assembly may be configured to be disposed on the outside of the respective right and left leg of the user in deployment of the leg harness on the user.

In another aspect, multiple, securable leg straps may be provided on the frame of each of the right harness assembly and the left harness assembly. The leg straps may facilitate securement of the right harness assembly and the left harness assembly to the respective right and left legs of the user.

In another aspect, a cushioned pad may be provided on the frame at each corresponding leg strap to cushion the leg of the user against the frame.

In another aspect, the frame of each harness assembly may include a thigh portion. A calf portion may extend from the thigh portion. The hinge may pivotally connect the calf portion to the thigh portion. The thigh portion and the calf portion may be configured for securement on the calf and thigh, respectively, on the corresponding leg of the user.

In another aspect, the frame of each harness assembly may include a foot portion which extends from the calf portion. The foot portion may be configured for securement on the foot on the corresponding leg of the user.

In another aspect, at least one foot strap may be provided on the foot portion.

In another aspect, the thigh portion of each frame may include a lower end, an upper end, a front edge, a rear edge, an outer surface and an inner thigh portion surface.

In another aspect, the calf portion of each frame may include an upper end, a lower end, a front edge, a rear edge, an outer surface and an inner surface. The hinge may pivotally attach the upper end of the calf portion to the lower end of the thigh portion.

In another aspect, the leg straps may be attached to the front edge and the rear edge of the thigh portion and to the front edge and the rear edge of the calf portion of each frame.

In another aspect, the cushioned pads may be provided on the inner thigh portion surface of the thigh portion and on the inner surface of the calf portion.

In another aspect, at least one magnet may be provided on the outer surface of the thigh portion.

In another aspect, the foot portion may extend from the lower end of the calf portion.

In another aspect, the foot portion may be C-shaped with a bottom segment extending from the lower end of the calf portion and an inner segment extending from the bottom segment.

In another aspect, at least one climbing spur may be provided on the inner segment of the foot portion.

In another aspect, at least one spur mount opening may be provided in the inner segment of the foot portion. The climbing spur may be attachable to the inner segment via the spur mount opening.

In another aspect, the hinge may include a hinge head at the upper end of the calf portion of the frame, a toothed, first disk on the hinge head, a toothed, second disk on the lower end of the thigh portion, and a spring engaging the second disk. The spring may normally bias the second disk into meshing relationship with the first disk to secure the calf portion at a selected angle with respect to the thigh portion of the frame. The second disk may be selectively disengaged from the first disk against the bias imparted by the spring to facilitate pivoting of the calf portion with respect to the thigh portion.

In another aspect, the hinge locking mechanism may include a hinge locking disk in the hinge. The hinge locking arm may be configured to slidably engage the hinge locking disk in the locking position and to slidably disengage the hinge locking disk in the unlocking position.

In another aspect, the hinge locking arm of the hinge locking mechanism may include an elongated shaft. A tab may be provided on the shaft. The tab may be digitally grasped to facilitate selectively deployment of the hinge locking arm in the locking and unlocking positions.

In another aspect, a tab cavity may be provided in the thigh portion of the frame. The tab cavity may be sized and configured to accommodate the tab on the shaft of the hinge locking arm.

In another aspect, the hinge locking disk may include multiple disk teeth. Multiple teeth notches may be provided in the shaft of the hinge locking arm. The teeth notches in the shaft of the hinge locking arm may be suitably sized and configured to receive respective disk teeth on the hinge locking disk in the locking position of the hinge locking arm.

In another aspect, the shaft of the hinge locking arm may be pivotally disposed with respect to the thigh portion of the frame. The hinge locking arm may be selectively pivotal in the locking and unlocking positions.

In another aspect, a pivot pin may extend transversely from the shaft of the hinge locking arm. The pivot pin may pivotally mount the shaft on the thigh portion of the frame.

In another aspect, each of the right harness assembly and the left harness assembly may include a pair of parallel, spaced-apart frames. The frames may have a single foot portion which connects the ankle portions of the frames. The frames may be configured for positioning on the respective outside or inside of each corresponding leg of the user in deployment of the leg harness on the user's legs.

These and other objects, features, and advantages of the present invention will become more readily apparent from the attached drawings and the detailed description of the preferred embodiments, which follow.

### Brief Description of the Drawings

The preferred embodiments of the invention will hereinafter be described in conjunction with the appended drawings provided to illustrate and not to limit the invention, where like designations denote like elements, and in which:
FIG. 1 presents perspective views of a right harness assembly and a left harness assembly configured for deployment on the respective right and left legs of a user (not illustrated) according to an illustrative embodiment of the leg harness and locking mechanism device of the present invention;
FIG. 2 presents a perspective view of the right and left harness assemblies, deployed on the respective right and left legs of the user in application of the leg harness and locking mechanism device;
FIG. 3 presents a side view of the leg harness and locking mechanism device illustrated in FIG. 2, deployed on the legs of the user, with the user ascending a wooden structure in application of the leg harness and locking mechanism device;
FIG. 4 presents a perspective view of the right and left harness assemblies deployed on the legs of the user and magnetic attachment of a pair of tools to a magnet on the right harness assembly;
FIG. 5A presents a perspective view of a hinge locking mechanism which includes a pivoting hinge locking arm disengaging the hinge on the frame of each harness assembly in the locking position of the hinge locking mechanism;
FIG. 5B presents a perspective view of the hinge locking mechanism illustrated in FIG. 5A, with the pivoting hinge locking arm engaging the hinge on the frame of each harness assembly in the unlocking position of the hinge locking mechanism;
FIG. 6A presents a perspective view of an alternative hinge locking mechanism which includes a slidable hinge locking arm disengaging a hinge locking disk on the hinge of the frame of each harness assembly in the unlocking position of the hinge locking mechanism;
FIG. 6B presents a perspective view of the hinge locking mechanism illustrated in FIG. 6A, with the slidable hinge locking arm engaging the hinge locking disk on the hinge of the frame of each harness assembly in the locking position of the hinge locking mechanism;
FIG. 7 presents a right harness assembly and a left harness assembly configured for deployment on the respective right and left legs of a user (not illustrated) according to an alternative illustrative embodiment of the leg harness and locking mechanism device of the present invention;
FIG. 8 is a perspective view of the right and left harness assemblies illustrated in FIG. 1; and
FIG. 9 is an exploded perspective view illustrating attachment of a pair of climbing spurs to a foot portion on the frame of each harness assembly.

Like reference numerals refer to like parts throughout the several views of the drawings.

### Detailed Description

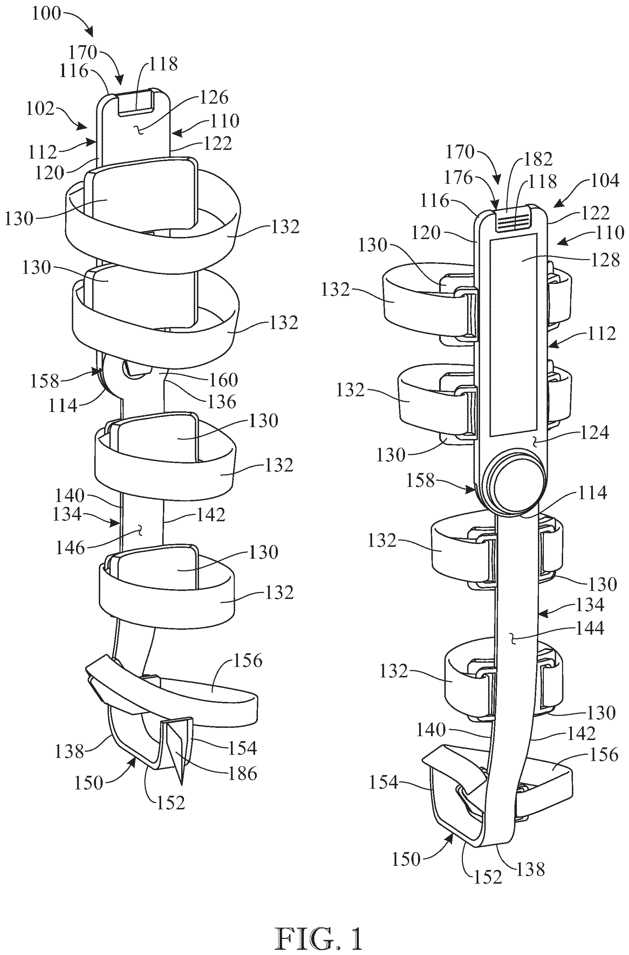

The following detailed description is merely exemplary in nature and is not intended to limit the described embodiments or the application and uses of the described embodiments. As used herein, the word "exemplary" or "illustrative" means "serving as an example, instance, or illustration." Any implementation described herein as "exemplary" or "illustrative" is not necessarily to be construed as preferred or advantageous over other implementations. All of the implementations described below are exemplary implementations provided to enable persons skilled in the art to make or use the embodiments of the disclosure and are not intended to limit the scope of the disclosure, which is defined by the claims. For purposes of description herein, the terms "upper", "lower", "left", "rear", "right", "front", "vertical", "horizontal", and derivatives thereof shall relate to the invention as oriented in FIG. 1. Furthermore, there is no intention to be bound by any expressed or implied theory presented in the preceding technical field, background, brief summary or the following detailed description. It is also to be understood that the specific devices and processes illustrated in the attached drawings, and described in the following specification, are simply exemplary embodiments of the inventive concepts defined in the appended claims. Hence, specific dimensions and other physical characteristics relating to the embodiments disclosed herein are not to be considered as limiting, unless the claims expressly state otherwise.

Shown throughout the figures, the present invention is directed toward a leg harness and locking mechanism device which can be attached to a leg of a user and locked at one or both of the user's knee or knees to relieve pressure on the knees during work.

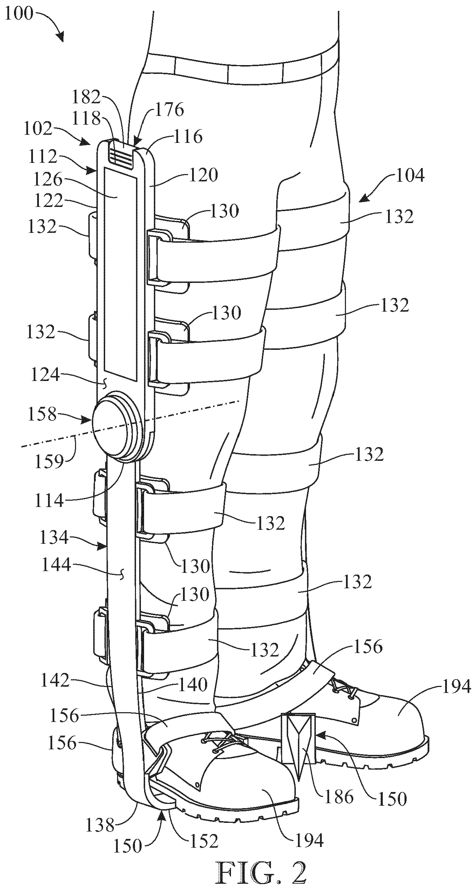

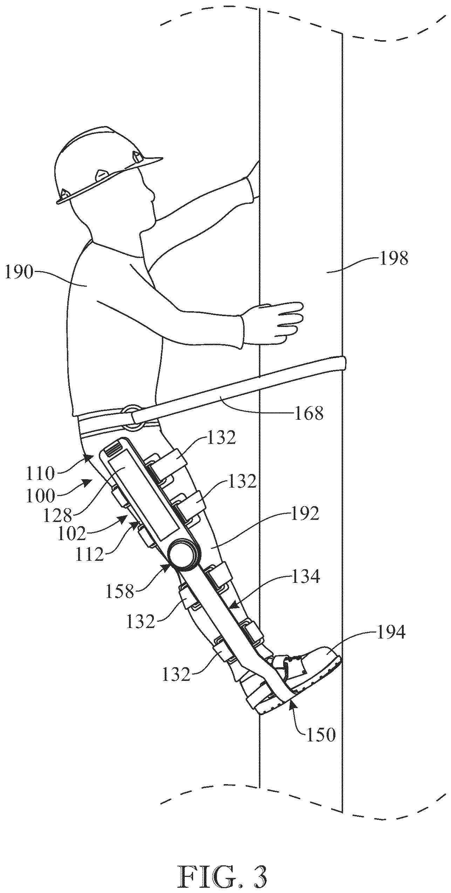

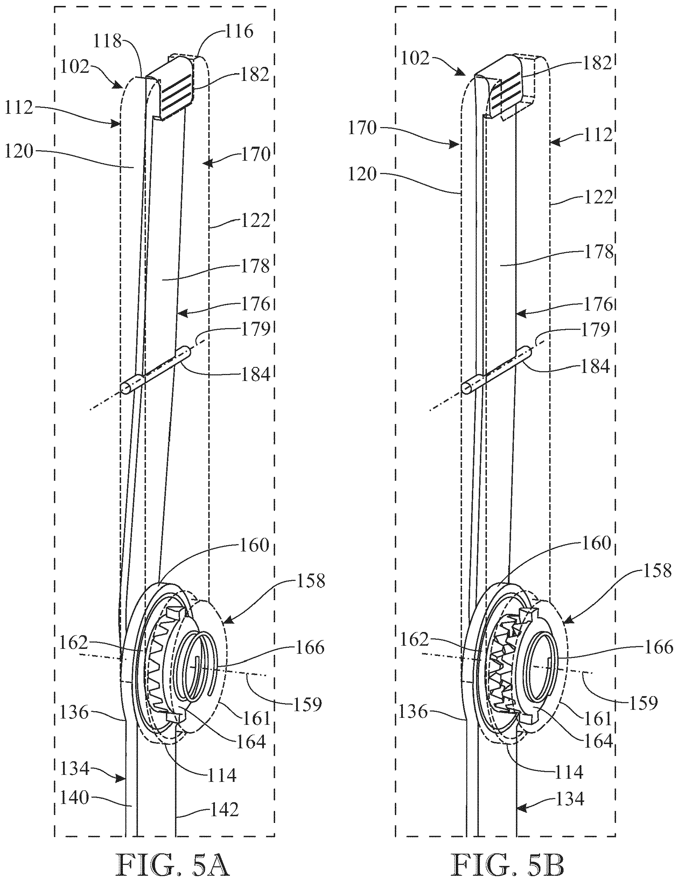

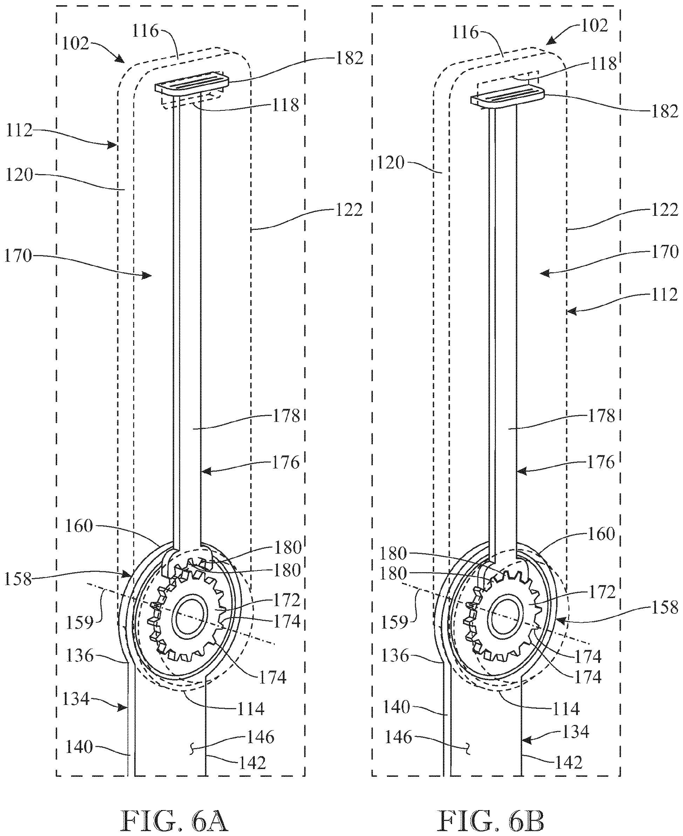

Referring initially to FIGS. 1-6B, 8 and 9, a leg harness and locking mechanism device 100 is illustrated in accordance with a first exemplary embodiment of the present invention. As shown for instance in FIG. 1, the leg harness and locking mechanism device 100 may include at least one harness assembly 102, 104. As illustrated in FIG. 2, each harness assembly 102, 104 of the leg harness and locking mechanism device 100 may be suitably configured for deployment on at least one leg 192 of a user 190 in preparation for climbing a tree, utility pole, or other wooden structure 198 (FIG. 3). Each harness assembly 102, 104 may include a hinge 158 which facilitates pivoting or folding of the harness assembly 102, 104 at the corresponding knee of the user 190, about a hinge rotation axis 159. The harness assembly 102, 104 may have a hinge locking mechanism 170, shown in further detail in FIGS. 5A-5B and 6A-6B, which is selectively operable to unlock the hinge 158 and allow the harness assembly 102, 104 to fold or pivot at the knee or knees of the user 190, and lock the hinge 158 to prevent the harness assembly 102, 104 from pivoting or folding, such as to maintain the harness assembly 102, 104 in a straight position. Accordingly, the locked hinge 158 may relieve pressure on the user's knee or knees.

In some embodiments, the same harness assembly 102, 104 may be configured for use as the right harness assembly 102 for deployment on the right leg 192 of the user 190 or as the left harness assembly 104 for deployment on the left leg 192 of the user 190. In some embodiments, the right harness assembly 102 and the left harness assembly 104 may be configured for deployment on the respective right and left legs 192 of the user 190. In some embodiments, each harness assembly 102, 104 may be configured for deployment on the outside or inside of the corresponding leg 192 of the user 190.

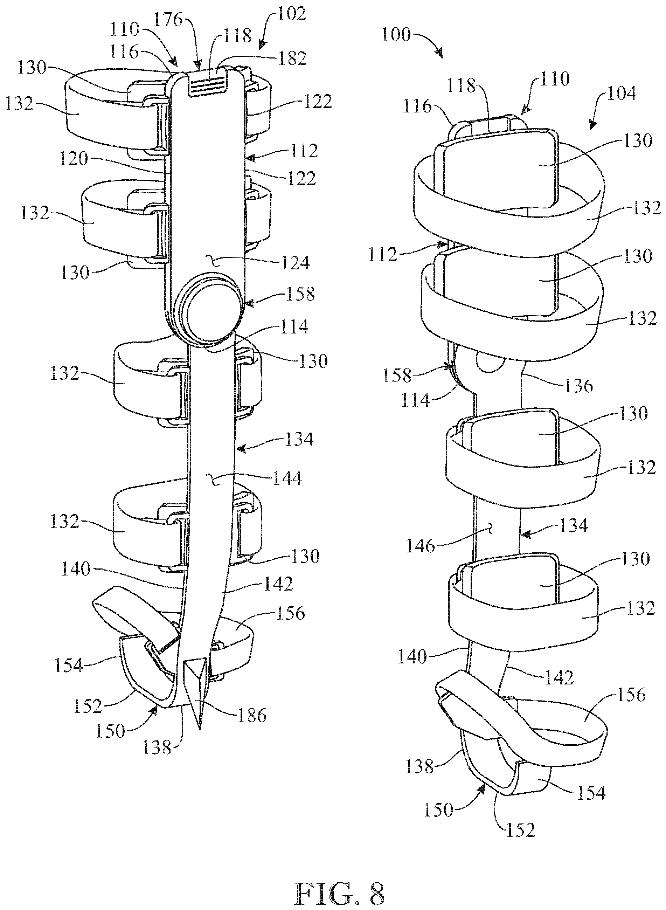

Referring to FIGS. 1 and 8, in some embodiments, each of the right harness assembly 102 and the left harness assembly 104 may include a frame 110, which may be generally rigid and provide a structural robustness to the harness assembly 102, 104. The frame 110 may be securable on the corresponding right or left leg 192 of the user 190. The hinge 158 may facilitate pivoting of the frame 110 at the knee of the user 190. The frame 110 of the right harness assembly 102 and the left harness assembly 104 may be configured to be disposed on the inside or outside of the respective right and left legs 192 of the user in deployment of the leg harness and locking mechanism device 100 on the user 190. For example, the illustration of FIG. 1 shows an embodiment in which the frames 110 of the right and left harness assemblies 102 and 104 are configured to extend along the outside of the legs 192, whereas the illustration of FIG. 8 shows an embodiment in which the frames 110 of the right and left harness assemblies 102 and 104 are configured to extend along the inside of the legs 192.

The frames 110 of the right harness assembly 102 and the left harness assembly 104 may be securable on the respective right and left legs 192 of the wearer 190 according to any suitable technique known by those skilled in the art. In some embodiments, multiple, securable leg straps 132 may be provided on the frame 110 of each harness assembly 102, 104. The leg straps 132 may facilitate securement of the right harness assembly 102 and the left harness assembly 104 to the respective right and left legs 192 of the user 190, for instance using hook and loop fasteners, buckles, and/or other suitable fastening mechanism known by those skilled in the art in order to secure the leg strap 132 in a looped or wrapped-around configuration about the corresponding leg 192, as shown in FIG. 2.

With continued reference to FIG. 1, in some embodiments, a cushioned pad 130 may be provided at each corresponding leg strap 132. The cushioned pads 130 may be configured to cushion the corresponding leg 192 of the user 190 against the frame 110 when the leg harness and locking mechanism device 100 is deployed on the user's leg or legs 192. In some embodiments, such as the present embodiment, the cushioned pads 130 may be oriented towards the leg 192 and configured to contact and abut against the leg 192. In some embodiments, as shown, the cushioned pads 130 may be affixed to the frame 110.

In some embodiments, the frame 110 of each harness assembly 102, 104 may include a thigh portion 112. A calf portion 134 may extend from the thigh portion 112. The hinge 158 may pivotally connect the calf portion 134 to the thigh portion 112 about the hinge rotation axis 159. The thigh portion 112 and the calf portion 134 may be configured for securement on the calf and thigh, respectively, on the corresponding leg 192 of the user 190.

In some embodiments, the frame 110 of each harness assembly 102, 104 may include a foot portion 150 which extends from the calf portion 134, and more preferably, from a bottom end of the calf portion 134 opposite to the hinge 158. The foot portion 150 may be configured for securement on the foot 194 on the corresponding leg 192 of the user 190. In some embodiments, at least one foot strap 156 may be provided on the foot portion 150 to facilitate securement of the foot portion 150 to the user's foot 194.

With continued reference to FIG. 1, in some embodiments, the thigh portion 112 of each frame 110 may include a lower end 114, an upper end 116, a front edge 120, a rear edge 122, an outer surface 124 configured to face away from the user's thigh and an inner surface 126 configured to face towards the user's thigh. The calf portion 134 of each frame 110 may include an upper end 136, a lower end 138, a front edge 140, a rear edge 142, an outer surface 144 configured to face away from the user's calf, and an inner surface 146 configured to face towards the user's calf. The hinge 158 may pivotally attach the upper end 136 of the calf portion 134 to the lower end 114 of the thigh portion 112. The foot portion 150 may extend from the lower end 138 of the calf portion 134. In turn, a tab cavity 118 may be provided in the upper end 116 of the thigh portion 112, for purposes described hereinafter.

In some embodiments, the leg straps 132 may be attached to the front edge 120 and the rear edge 122 of the thigh portion 112 and to the front edge 140 and the rear edge 142 of the calf portion 134 of each frame 110. The cushioned pads 130 may be provided on the inner surface 126 of the thigh portion 112 and on the inner surface 146 of the calf portion 134.

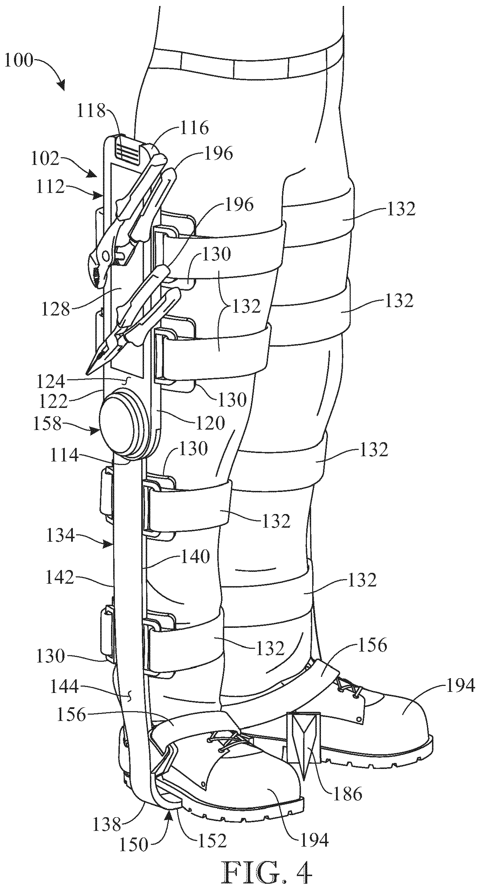

As illustrated in FIGS. 1-4, in some embodiments, at least one magnet 128 may be provided on the frame 110, and more preferably, at the thigh portion 112 of the frame 112, such as on the outer surface 124 of the thigh portion 112. As illustrated in FIG. 4, the magnet 128 may be configured to magnetically support one or more tools 196 in application of the leg harness and locking mechanism device 100. For instance, the magnet 128 may be of sufficient size to allow a majority of a metallic working part of the tool 196 to secure to the magnet 128, as shown.

As mentioned heretofore, the foot portion 150 of the frame 110 may extend from the lower end 138 of the calf portion 134. In some embodiments, the lower end 138 of the calf portion 134 and the foot portion 150 may form a C-shaped arrangement with a bottom segment 152 extending at an angle from the lower end 138 of the calf portion 134 and an inner segment 154 extending at an angle from the bottom segment 152. The bottom segment 152 and inner segment 154 may form an upwardly-directed L-shaped arrangement.

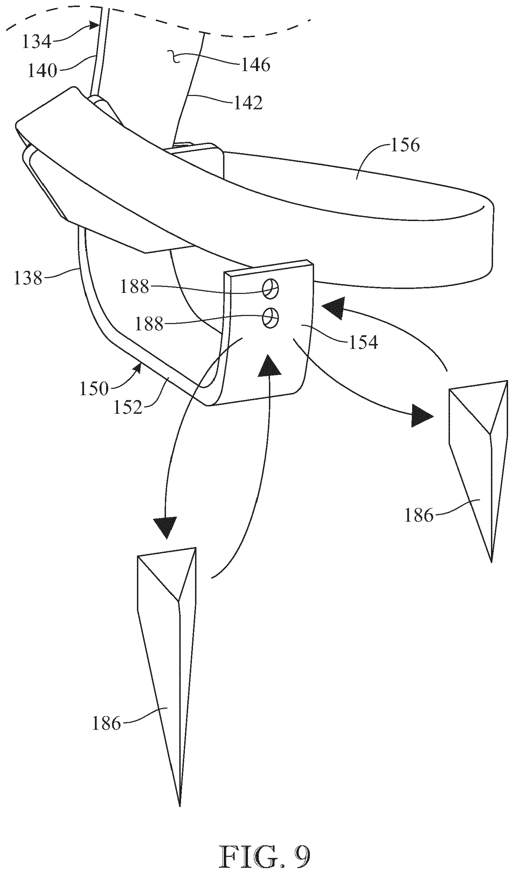

In some embodiments, at least one climbing spur 186 may be provided on the foot portion 150, such as on the inner segment 154 of the foot portion 150. Each climbing spur 186 may be fixedly or detachably attached to the foot portion 150 according to the knowledge of those skilled in the art. In a non-limiting example, illustrated in FIG. 9, at least one spur mount opening 188 may be provided in the inner segment 154 of the foot portion 150. The climbing spur 186 may be attachable to the inner segment 154 via the spur mount opening 188 such as by a snap-fastener, bolt and nut fastener, screw, or other fastener configured to attach to the spur mount opening 188.

The hinge 158 on the frame 110 of each harness assembly 102, 104 may have any design which is suitable for the purpose of facilitating pivoting or bending of the thigh portion 112 and the calf portion 134 relative to each other about the hinge rotation axis 159. As illustrated in FIGS. 5A, 5B, 6A and 6B, in some embodiments, the hinge 158 may include a hinge head 160 at the upper end 136 of the calf portion 134 of the frame 110, and a hinge head 161 at the lower end 114 of the thigh portion 112 of the frame 110 (it should be noted that the thigh portion 112 shown in phantom lines 112), the hinge heads 160, 161 pivotably connected to one another about the hinge rotation axis 159.

With respect to the hinge locking mechanism 170, the illustrations of FIGS. 5A and 5B show a first embodiment of the hinge locking mechanism 170, while the illustrations of FIGS. 6A and 6B show a second embodiment of said hinge locking mechanism 170.

With reference initially to FIGS. 5A and 5B, the hinge locking mechanism 170 may include a hinge locking arm 176. The hinge locking arm 176 may include an elongated shaft 178, which may extend generally from the upper end 116 of the thigh portion 116 of the frame 110 to the hinge 158. A pivot pin 184 may extend transversely from an intermediate section of the shaft 178. The pivot pin 184 may pivotally mount the shaft 178 on the thigh portion 112 of the frame 110. The shaft 178 may pivot relative to the frame 110 about an arm rotation axis 179 defined by the pivot pin 184, the arm rotation axis 179 arranged in a front-to-back direction generally perpendicular to the hinge rotation axis 159. As further shown, a tab 182 may be provided on the upper end of the shaft 178. The tab 182 may be configured to be at least partially received within the tab cavity 118 of the thigh portion 112. As further shown, a toothed, first disk 162 may be provided on the hinge head 160 of the upper end 136 of the calf portion 134. A toothed, second disk 164 may be carried by the shaft 178 at a bottom end thereof, the second disk 164 positioned adjacent to the hinge head 161 of the lower end 114 of the thigh portion 112 and adjacent to the first disk 162. The second disk 164 may be jointly pivotable with the shaft 178 about the arm rotation axis 179. A spring 166 (e.g., a compression spring) may engage the second disk 164 and the hinge head 161 of the thigh portion 112. The spring 166 may normally bias the second disk 164 away from the hinge head 161 of the thigh portion 112 and into meshing relationship with the first disk 162 to switch the hinge locking mechanism 170 to the locking position (FIG. 5A).

In operation of the locking mechanism 170, the tab 182 may be digitally grasped, pushed, or otherwise manipulated to facilitate selectively deployment of the hinge locking arm 176 in a locking position (FIG. 5A) and unlocking (FIG. 5B) position. In the locking position, the hinge locking arm 176 is pivoted upward about the arm rotation axis 179 such that the upper end of the hinge locking arm 176 (carrying tab 182) is shifted towards the tab cavity 118 of the thigh portion 112 and the lower end of the hinge locking arm 176 (carrying the second disk 164) is shifted away from the hinge head 161 of the thigh portion 112 and towards the first disk 162, and the second disk 164 meshes with the first disk 162. As a result of the first and second disks 162, 164 engaging with each other, the hinge locking arm 176 is unable to rotate about the hinge rotation axis 159 with respect to the calf portion 134 of the frame 110. In turn, because the hinge locking arm 176 is secured to the thigh portion 112 of the frame 110 at the pivot pin 182, the thigh portion 112 is non-rotationally engaged with the hinge locking arm 176 with respect to the hinge rotation axis 159; in consequence, in locking the hinge locking arm 176 with respect to the calf portion 134, the thigh portion 112 is also prevented from rotating with respect to the calf portion 134 about the hinge rotation axis 159. It should be noted that, in some embodiments, such as the present embodiment, the tab 182 may be received within the tab cavity 118 in the locking configuration, and thereby enhance the non-rotational relationship between the hinge locking arm 176 and the thigh portion 112. In the locking position, the hinge locking arm 176 thus locks the hinge 158 to secure the calf portion 134 at a selected angle with respect to the thigh portion 112 of the frame 110, such as to maintain the harness assembly 102, 104 in the straight position and prevent the harness assembly 102, 104 from pivoting or folding at the knee of the user 190. The locked hinge 158 may relieve pressure on the user's knee or knees. The spring 166 may maintain the hinge locking mechanism 170 in the locking position in the absence of user operation of the tab 182.

In order to switch to the unlocking position, the user may press the locking tab 182 inward, as shown in FIG. 5B, counteracting the bias of the spring 166. Such action causes the hinge locking arm 176 to pivot downward about the arm rotation axis 179 as shown. As a result, the bottom end of the hinge locking arm 176, including the second disk 164 carried at said bottom end, is pivoted towards the hinge head 161 of the thigh portion 110, causing the second disk 164 to disengage from the first disk 162 and the calf portion 134 to thereby become freed for rotation about the hinge rotation axis 159 with respect to the non-rotationally related (with respect to hinge rotation axis 159), hinge locking arm 176 and thigh portion 110. In the unlocking position, the hinge locking mechanism 170 thus facilitates folding or pivoting of the harness assembly 102, 104 at the knee or knees of the user 190.

In a second example, as illustrated in FIGS. 6A and 6B, the hinge locking mechanism 170 may include a hinge locking disk 172 in the hinge 158. The hinge locking disk 172 may be non-rotationally carried by the hinge head 160 at the upper end 136 of the calf portion 134 of the frame 110. The hinge locking arm 176 may slidably carried by the thigh portion 110 of the frame 110, and may be configured to slidably engage the hinge locking disk 172 in the locking position (FIG. 6B) and to slidably disengage the hinge locking disk 172 in the unlocking position (FIG. 6A). In order to slide the hinge locking arm 176, the user may operate the tab 182 by sliding the tab 182 upward or downward along the tab cavity 118 of the thigh portion 110, as shown. The hinge locking disk 172 may include multiple disk teeth 174. Multiple teeth notches 180 may be provided in the shaft 178 of the hinge locking arm 176. The teeth notches 180 in the shaft 178 of the hinge locking arm 176 may be suitably sized and configured to receive the respective disk teeth 174 on the hinge locking disk 172 in the locking position of the hinge locking arm 176. The shaft 178 of the hinge locking arm 176 may thus be configured to engage the hinge locking disk 172 in the hinge 158 to selectively lock the hinge 158 in the locking position and disengage the hinge locking disk 172 in the unlocking position. In other embodiments, the shaft 178 may be configured to engage and disengage some other component or components in the hinge 158 for the respective locking and unlocking of the hinge 158.

In an illustrative application of the leg harness and locking mechanism device 100, as shown in FIG. 2, the right harness assembly 102 and the left harness assembly 104 may be deployed in place on the respective right and left legs 192 of the user 190 in preparation for the user's ascending a tree, utility pole, or other wooden structure 198 (FIG. 3). Accordingly, with the thigh portion 112 and the calf portion 134 disposed outside or inside the corresponding leg 192, the feet 194 of the user 190 may be placed in the respective foot portions 150 of the frames 110. The leg straps 132 may be fastened around the thigh and calf of each corresponding leg 192. The foot straps 156 may be secured to fasten the user's feet 194 in the respective foot portions 150. As illustrated in FIG. 9, one or more of the climbing spurs 186 may be attached to the inner segment 154 of each foot portion 150. In some applications, one or more tools 196 may be magnetically attached to the magnet 128 on the thigh portion 112 of each frame 110, as illustrated in FIG. 4.

As illustrated in FIG. 3, a waist strap 168 may be secured around the waist of the user 190 and around the wooden structure 198. With help of the leg harness and locking mechanism device 100, the user 190 may alternately apply and release the climbing spurs 186 against the respective sides of the wooden structure 198 to ascend the wooden structure 198. After the user 190 has attained the desired height on the wooden structure 198, the hinge locking mechanisms 170 may be deployed from the unlocking position (FIG. 5B or 6A) to the locking position (FIG. 5A or 6B) to lock the hinge 158 of each harness assembly 102 at the knee on the corresponding leg 192 of the wearer 190. This expedient may release the holding pressure which is required for the user 190 to remain in the elevated position on the wooden structure 198 from the knees of the user 190 throughout the time which is necessary for the user 190 to complete the work on the wooden structure 198. To descend the wooden structure 198, the user 190 may release the hinge locking mechanism 170 (FIG. 5B or 6A) to facilitate pivoting of the frames 110 at the knees on the respective legs 192 of the wearer 190. After use, the harness assemblies 102 and 104 may be removed from the user's respective legs 192 by unfastening the leg straps 132 and the foot straps 156.

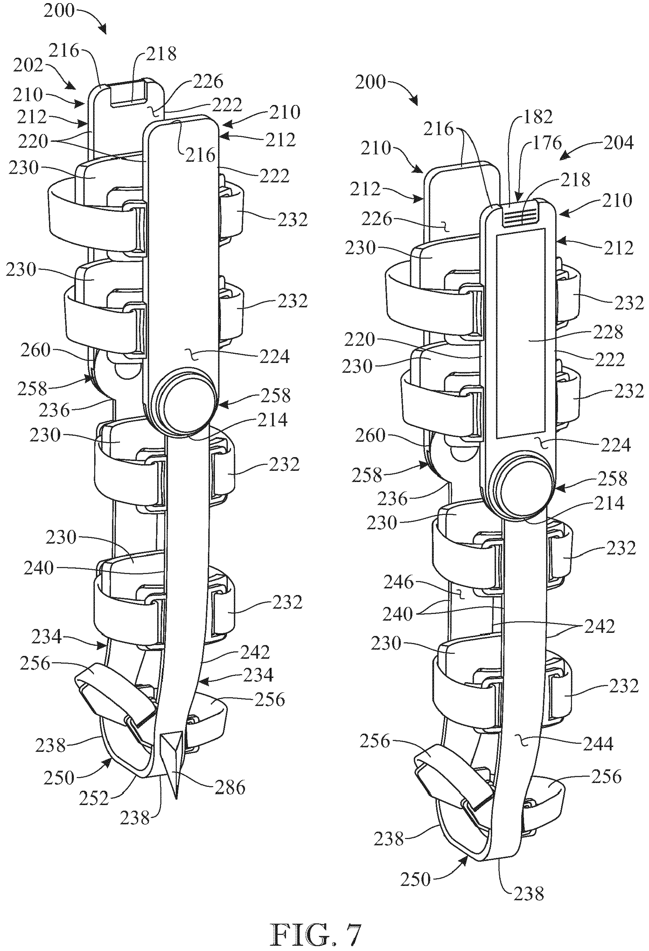

Referring next to FIG. 7, an alternative illustrative embodiment of the leg harness is generally indicated by reference numeral 200. In the leg harness 200, elements which are analogous to the respective elements of the leg harness and locking mechanism device 100 that was heretofore described with respect to FIGS. 1-6, 8, and 9 are designated by the same respective numerals in the 200-299 series in FIG. 7. Each of the right harness assembly 202 and the left harness assembly 204 may include a pair of parallel, spaced-apart frames 210. The frames 210 may have a single foot portion 250 which connects the ankle portions 234 of the frames 210, such that the frames 210 and foot portion 250 form a generally U-shaped arrangement. The frames 210 may be configured for positioning on the respective outside and inside of each corresponding leg 192 (FIG. 3) of the user 190 in deployment of the leg harness 200 on the user's legs 192. The straps 232, 256 may extend between the frames 210 and may secure the frames 210 at said opposite position, outside and inside of the leg 192. Cushioned pads 230 may be provided in pairs, with each pair of cushioned pads 230 facing one another and carried by the frames 210 at a respective, discrete position therealong. Application of the leg harness 200 may be as was heretofore described with respect to the leg harness and locking mechanism device 100.

Since many modifications, variations, and changes in detail can be made to the described preferred embodiments of the invention, it is intended that all matters in the foregoing description and shown in the accompanying drawings be interpreted as illustrative and not in a limiting sense. Thus, the scope of the invention should be determined by the appended claims and their legal equivalents.

## Claims

1. A leg harness and locking mechanism device, comprising:
at least one harness assembly, each harness assembly of said at least one harness assembly deployable along and securable to one of a left leg and a right leg of a user, wherein each harness assembly comprises one or more frames deployable along and securable to respective one or more sides of said one of the left leg and the right leg, wherein each frame of the one or more frames comprises:
a thigh portion and a calf portion, respectively deployable along a thigh and a calf of said one of the left leg and the right leg,
a hinge, configured to facilitate a relative pivoting between the thigh portion and the calf portion about a hinge rotation axis to thereby enable a folding of said each frame at a knee of said one of the left leg and the right leg, and
a hinge locking mechanism configured to selectively engage the hinge, the hinge locking mechanism operable to engage the hinge and prevent said relative pivoting and to alternatively disengage the hinge and enable said relative pivoting.

2. The leg harness and locking mechanism device of claim 1, wherein said each harness assembly is configured to adopt at least one locked configuration, wherein, in each locked configuration of the at least one locked configuration, the thigh and calf portions of each frame of the one or more frames of said each harness assembly are arranged at a respective pivoting angle relatively to one another about the hinge rotation axis of said each frame, and the hinge locking mechanism of said each frame is arranged engaging the hinge of said each frame and preventing said relative pivoting.

3. The leg harness and locking mechanism device of claim 2, wherein said at least one locked configuration comprises two or more locked configurations, wherein the respective pivoting angles of the two or more locked configurations are different from one another, in particular wherein the hinge comprises a toothed disk, and the hinge locking mechanism comprises a notched member, the notched member configured to mesh with the toothed disk at two or more different angular positions relative to one another about the hinge rotation axis to define said different, respective pivoting angles.

4. The leg harness and locking mechanism device of claim 2, wherein the thigh portion and the calf portion of said each frame are generally rigid and configured to relieve pressure on said knee in said at least one locked configuration.

5. The leg harness and locking mechanism device of claim 1, wherein said each frame further comprises a foot portion, the foot portion extending from the calf portion of said each frame, the foot portion deployable at and securable to the foot of said one of the left leg and the right leg, and in particular, wherein the foot portion and the calf portion of said each frame form a generally L-shaped arrangement, the foot portion configured to extend below the foot and enable the foot to step on the foot portion, or, wherein each harness assembly comprises one or more climbing spurs.

6. The leg harness and locking mechanism device of claim 1, wherein said one or more frames comprise a left frame and a right frame deployable along respective left and right sides of said one of the left leg and the right leg.

7. The leg harness and locking mechanism device of claim 6, wherein said one or more frames further comprise a foot portion, the foot portion extending from the respective calf portions of the left and right frames, the foot portion deployable at and securable to the foot of said one of the left leg and the right leg, and in particular wherein the foot portion and calf portions form a generally U-shaped arrangement, the foot portion configured to extend below the foot and enable the foot to step on the foot portion.

8. The leg harness and locking mechanism device of claim 1, wherein each frame of the one or more frames of said each harness assembly further comprises at least one cushioning pad deployable between said each frame and said one of the left leg and the right leg.

9. The leg harness and locking mechanism device of claim 1, wherein each harness assembly further comprises a plurality of straps configured to extend about said one of the left leg and the right leg and secure the one or more frames of said each harness assembly to said one of the left leg and the right leg, and in particular wherein said plurality of straps comprises at least one first strap configured to secure the thigh portion to the thigh and at least one second strap configured to secure the calf portion to the calf.

10. The leg harness and locking mechanism device of claim 1, wherein the hinge locking mechanism of said each frame comprises an elongate, hinge locking arm extending along the thigh portion of said each frame, from an upper area of said thigh portion to a lower area of said thigh portion, wherein the hinge locking arm is operable at said upper area of said thigh portion to cause the hinge locking arm to selectively engage the hinge at said lower area of the thigh portion to alternatively engage the hinge and prevent said relative pivoting or disengage the hinge and enable said relative pivoting.

11. The leg harness and locking mechanism device of claim 10, wherein the upper area of the thigh portion is arranged at an upper end of said thigh portion.

12. The leg harness and locking mechanism device of claim 10, wherein the hinge locking arm is pivotably connected to an intermediate area of said thigh portion, said intermediate area arranged between said upper and lower areas of said thigh portion, wherein the hinge locking arm is selectively pivotable towards and away from said hinge at said lower area responsively to a manual pivoting of said hinge locking arm away from and towards said upper area of said thigh portion, respectively, to engage the hinge and prevent said relative pivoting and to alternatively disengage the hinge and enable said relative pivoting.

13. The leg harness and locking mechanism device of claim 10, wherein the hinge locking arm is slidably connected to said thigh portion and is selectively slidable with respect to said thigh portion towards and away from said hinge to engage the hinge and prevent said relative pivoting and to alternatively disengage the hinge and enable said relative pivoting.

14. A leg harness and locking mechanism device, comprising:
at least one harness assembly, each harness assembly of said at least one harness assembly deployable along and securable to one of a left leg and a right leg of a user, wherein each harness assembly comprises one or more frames deployable along and securable to respective one or more sides of said one of the left leg and the right leg, wherein each frame of the one or more frames comprises:
a thigh portion and a calf portion, the thigh and calf portions generally rigid and respectively deployable along a thigh and a calf of said one of the left leg and the right leg,
a hinge, configured to facilitate a relative pivoting between the thigh portion and the calf portion about a hinge rotation axis to thereby enable a folding of said each frame at a knee of said one of the left leg and the right leg, and
a hinge locking mechanism configured to selectively engage the hinge, the hinge locking mechanism comprising an elongate, hinge locking arm extending along the thigh portion of said each frame, from an upper area of said thigh portion to a lower area of said thigh portion, wherein the hinge locking arm is operable at said upper area of said thigh portion to cause the hinge locking arm to selectively engage the hinge at said lower area of the thigh portion to alternatively engage the hinge and prevent said relative pivoting or disengage the hinge and enable said relative pivoting; wherein
said each harness assembly is configured to adopt at least one locked configuration, wherein, in each locked configuration of the at least one locked configuration, the thigh and calf portions of each frame of the one or more frames of said each harness assembly are arranged at a respective pivoting angle relatively to one another about the hinge rotation axis of said each frame, and the hinge locking mechanism of said each frame is arranged engaging the hinge of said each frame and preventing said relative pivoting, the thigh portion and calf portion configured to relieve pressure on said knee.

15. A leg harness and locking mechanism device, comprising:
at least one harness assembly, each harness assembly of said at least one harness assembly deployable along and securable to one of a left leg and a right leg of a user, wherein each harness assembly comprises a plurality of straps and one or more frames, the one or more frames deployable along respective one or more sides of said one of the left leg and the right leg and securable to said one of the left leg and the right leg by said plurality of straps, wherein each frame of the one or more frames comprises:
a thigh portion and a calf portion, the thigh and calf portions generally rigid and respectively deployable along a thigh and a calf of said one of the left leg and the right leg,
a hinge, configured to facilitate a relative pivoting between the thigh portion and the calf portion about a hinge rotation axis to thereby enable a folding of said each frame at a knee of said one of the left leg and the right leg, and
a hinge locking mechanism configured to selectively engage the hinge, the hinge locking mechanism comprising an elongate, hinge locking arm extending along the thigh portion of said each frame, from an upper area of said thigh portion to a lower area of said thigh portion, wherein the hinge locking arm is operable at said upper area of said thigh portion to cause the hinge locking arm to selectively engage the hinge at said lower area of the thigh portion to alternatively engage the hinge and prevent said relative pivoting or disengage the hinge and enable said relative pivoting; wherein
said each harness assembly is configured to adopt at least one locked configuration, wherein, in each locked configuration of the at least one locked configuration, the thigh and calf portions of each frame of the one or more frames of said each harness assembly are arranged at a respective pivoting angle relatively to one another about the hinge rotation axis of said each frame, and the hinge locking mechanism of said each frame is arranged engaging the hinge of said each frame and preventing said relative pivoting, the thigh portion and calf portion configured to relieve pressure on said knee.
